# EUROPEAN PATENT SPECIFICATION

(11) **EP 2 612 671 B1**
(45) Date of publication and mention of the grant of the patent: **09.10.2019**
(21) Application number: 11821082.2
(22) Date of filing: 12.08.2011
(51) Int. Cl.: A61K 31/7048, A61K 47/18, A61K 9/08, A61K 9/19, A61P 9/10, A61K 9/00

(54) **A CLINICAL PREPARATION OF SCUTELLARIN AND THE PREPARATION METHOD THEREOF**
KLINISCHES PRÄPARAT AUS SCUTELLARIN UND HERSTELLUNGSVERFAHREN DAFÜR
PRÉPARATION CLINIQUE DE SCUTELLARINE ET PROCÉDÉ DE PRÉPARATION DE CELLE-CI

(30) Priority: 02.09.2010 CN 201010273274
(43) Date of publication of application: 10.07.2013
(73) Proprietor: KPC Pharmaceuticals, Inc., Kunming, Yunnan 650106 (CN)
(72) Inventor: ZHANG, Guoli, Kunming, Yunnan 650106 (CN); YANG, Zhaoxiang, Kunming, Yunnan 650106 (CN); ZHANG, Wei, Kunming, Yunnan 650106 (CN); PU, Junxue, Kunming, Yunnan 650106 (CN)
(74) Representative: Laufhütte, Dieter
(86) International application number: PCT/CN2011/078356
(87) International publication number: WO 2012/028056

(56) References cited:
- CN-A- 1 133 180
- CN-A- 1 424 031
- CN-A- 1 555 807
- CN-A- 101 993 462

## Description

### Field of the invention

The present invention relates to the field of pharmaceutics, more specifically to a clinical preparation of scutellarin and the preparation method thereof.

### Background of the invention

Breviscapine is isolated and extracted from Herba erigerontis which is the dried entire herb of *Erigeron breviscapus(Vant.)Hand-Mazz* (*Compositae*), and contains mainly scutellarin as well as a small amount of apigenin-7-O-glucronide and a mixture of various flavones, and has efficacies of promoting blood circulation for removing blood stasis, dispelling cold and relieving surperficies, relieving rigidity of muscles and activating collaterals, as well as dispelling pathogenic wind and removing dampness, and is commonly used clinically in the treatment of cardiovascular and cerebrovascular diseases. Scutellarin, also called breviscapine B, is the main ingredient of breviscapine, and also the main active pharmaceutical ingredient. Modern pharmacological studies suggest that scutellarin has good therapeutic effects on hypertension, cerebral hemorrhage, cerebral thrombosis, cerebral embolism polyneuritis, chronic arachnoiditis and sequelae thereof, and has certain therapeutic effects on rheumatism and coronary heart disease. Currently marketed preparations of scutellarin in China include common tablet, sustained-release tablet and injection, etc., wherein various treatments of injections are widely used due to their good therapeutic effect on cerebral thrombotic hemiplegia and coronary heart disease.

Scutellarin is a compound of flavonoid glycoside, and is water-insoluble in acidic conditions and easily water-soluble in alkaline conditions, thus the conventional techniques for preparing scutellarin (breviscapine) mostly employ solution of various alkalis (such as sodium hydroxide, potassium hydroxide, sodium bicarbonate, arginine, lysine, etc.) to dissolve scutellarin and then formulate it into preparations. Patent CN 200610010907 discloses formulating injections by employing water-soluble sodium salt of scutellarin formed by scutellarin and sodium bicarbonate. Patent CN 200610010923 and patent CN 200910095152 disclose formulating injections by employing water-soluble amino acid salt of scutellarin formed by scutellarin and L-arginine or L-lysine.

However, the basicity of the inorganic base salt or the alkaline amino acid salt is strong, thus the phenolic hydroxyl group of the scutellarin is readily to be oxidized, resulting in poor stability of scutellarin injection. In addition, small amount of flavonoids which are not flavone-glycoside mixed in the raw scutellarin will aggregate into flocculent sediment and precipitate during long-term refrigeration, resulting in an unqualified clarity. In order to solve this problem, in the prior art, plenty of inorganic base, organic base, organic acid is commonly added to form a stable buffer with a pH value below 8 as medium, or antioxidant, metal ion complexing agent and other excipients are added to solve the stability problem. The patents mentioned above formulate injectable preparations by employing an aqueous solution with a pH value of 7∼8, formed by L-arginine or L-lysine and glacial acetic acid, as stabilization system.

### Summary of the invention

In view of this, the purpose of the present invention is to provide a clinical solution preparation of scutellarin and the preparation method thereof, wherein said clinical preparation of scutellarin has good stable property, and said preparation method of the clinical preparation do not need to use buffer pair of organic acid and organic base to adjust a stable pH value.

To achieve the above-mentioned purpose, the present invention employs the following technical solutions:

A clinical solution preparation of scutellarin, comprising active ingredients and pharmaceutical excipients, wherein the active ingredient comprises scutellarin meglumine shown in formula (I), which is a new ionic compound obtained by salt-forming via ionic bond between the carboxyl group in scutellarin and the methylamino group in meglumine.

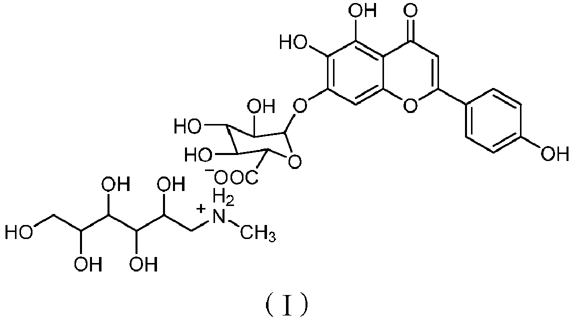

Meglumine is a common pharmaceutical excipient, which has a molecular formula of C₇H₁₇NO₅, molecular weight of 195.21, and is easily soluble in water, and has appropriate basicity and a certain extent of reducibility, and is safe and non-toxic. It can bind to a poorly water-soluble drug to improve the solubility of the drug in water (Mingsheng LUO, Complete collection of pharmaceutical excipients, Sichuan Science and Technology Press: 698-699), and can also complex with compounds containing phenolic hydroxyl group to protect the phenolic hydroxyl group from being oxidized.

For the clinical preparation of scutellarin according to the present invention, the amount of the active ingredient of the injection may be 0.01g/mL∼0.03 g/mL calculated on the basis of scutellarin, i.e., the injection of scutellarin may contain 0.01g ∼ 0.03g of the active ingredient scutellarin per milliliter.

The present invention provides a method for preparing the clinical preparation comprising the following steps:
Step 1: meglumine is added into a suspension of scutellarin and stirred, to form a clear solution, the molar ratio between scutellarin and meglumine is 1.0 : 1.0∼1.5;
Step 2: the solution obtained in step 1 is cooled to -2°C ∼ 1°C, and allowed to stand for more than 8 hours, filtered through 0.45 ∼ 0.8µm filter membrane, and the filtrate is collected;
Step 3: the filtrate obtained in step 2 is formulated into a clinical preparation of scutellarin by conventional means.

In the preparation method of the clinical preparation of scutellarin according to the present invention, by forming scutellarin meglumine salt shown in Formula (I) from the scutellarin and meglumine in step 1, the solubility of scutellarin in water can be improved, and the pH value of the solution is within the range of 6.5∼6.7, in which range the phenolic hydroxy group of scutellarin is very stable, and there is no need to use organic acid, organic alkaline buffering pair to adjust a stable pH value, thus the stability of the clinical preparation of scutellarin is improved.

The clarity of the scutellarin meglumine salt is a main index reflecting the stability thereof. It is found in the present invention by the clarity test of the scutellarin meglumine salt with different pH values that, when the pH value of the solution is 6.5∼6.7, the clarities are all good, whether it is stored in the state of solution for long term or redissolved after freeze drying; and when the pH value of the solution is greater than 7.0, clarity problem occurs at different time. When the molar ratio between scutellarin and meglumine is 1.0 : 1.0∼1.5, the pH of the solution is 6.5∼6.7 which is right the most stable pH value range. Wherein, preferably, the molar ratio between scutellarin and meglumine is 1.0:1.0.

In the preparation method of the clinical preparation of scutellarin according to the present invention, the solution obtained in step 1 is cooled to -2°C - 1°C in step 2, and is allowed to stand for more than 8 hours, filtered through 0.45∼0.8 µm filter membrane, and insoluble particles suspended in the solution other than scutellarin can be well precipitated on the bottom of the solution, and the supernatant can be filtered quickly, thus the production efficiency is greatly improved.

In the preparation method of the clinical preparation of scutellarin according to the present invention, the conventional means mentioned in step 3 is specifically as the follws: the filtrate obtained is adjusted to a required concentration by adding water for injection, to obtain a total solution of scutellarin injection, which is then adsorbed by activated charcoal, filtered, filled and sterilized, to obtain the aqueous injection of scutellarin.

In the method for preparing the clinical preparations of scutellarin according to the present invention, said adsorption by activated charcoal is an adsorption by activated charcoal for injection, specifically, activated charcoal for injection is added in 0.2%∼1.0% by weight of the total solution of scutellarin injection, and adsorbed for 30∼45 minutes.

In the preparation method of the clinical preparation of scutellarin according to the present invention, the filtration is two-step filtration, first filtered through 0.45 ∼ 0.8 µm filter membrane to remove the activated charcoal for injection, then filtered through 0.2 µm filter membrane to further remove bacteria by filtration.

The preparation method of the clinical preparation of scutellarin according to the present invention further comprises steps of determining the pH value of the scutellarin injection and the content of scutellarin.

The active ingredient of the clinical preparation of scutellarin according to the present invention comprises scutellarin meglumine, which improves the water solubility and stability of scutellarin. The appearance, clarity, visible foreign matter, pH value, content, related substances, and other indicators all comply with the relevant quality requirements, proved by stress test, accelerated test and long term stability test. The stability study on the compatibility with solutions of different pH values shows that the stability for compatibility of the clinical preparation of scutellarin according to the present invention is good, suggesting that the clinical preparation of scutellarin according to the present invention is stable in nature.

The preparation method of the clinical preparation of scutellarin according to the present invention does not need to use organic acid, organic alkaline buffering pairs to adjust a stable pH value, which reduces the use of antioxidants, metal ion complexing agents and other excipients, and avoids the possibility of developing adverse effects brought by excipients to the maximal extent, thus has a high safety. In addition, the preparation method of the clinical preparation of scutellarin according to the present invention accelerates the filtration speed in the production process, and greatly improves production efficiency.

### Detailed embodiments

To further understand the present invention, the present invention will be described in conjunction with examples described below, however, it should be appreciated that these descriptions are only for the further illustration of the features and advantages of the present invention, rather than limiting the scope of the claims of the present invention.

The effects of the present invention will be illustrated by specific examples below, however, the protection scopes of the present invention are not limited by the following examples.

The examples of the present invention disclose a clinical preparation of scutellarin and the preparation method thereof. Those skilled in the art can achieve them by referring to the contents disclosed herein, in combination with appropriate modifications of technological parameters. Particularly, all the similar replacement and modifications are obvious to those skilled in the art, which are all deemed to be included in the present invention. The product and method of the present invention have been described by preferred examples. It is apparent that the product and method described herein can be modified or altered appropriately and combined by those skilled in the art to achieve and apply the technique of the present invention, without departing from the content, spirit and scope of the present invention.

To achieve the purpose of the present invention, the present invention employs the following technical solutions:

A clinical preparation of scutellarin, comprising active ingredients and pharmaceutical excipients, wherein the active ingredient comprises scutellarin meglumine shown in formula (I), which is a new ionic compound obtained by salt-forming via ionic bond between the carboxyl group in scutellarin and the methylamino group in meglumine.

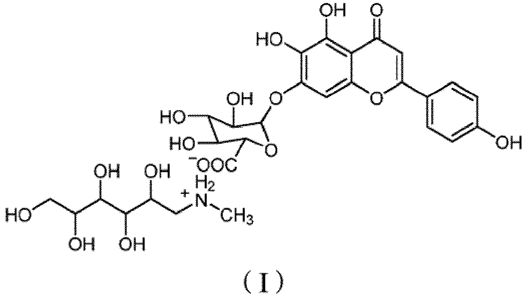

Meglumine is a common pharmaceutical excipient, which has a molecular formula of C₇H₁₇NO₅, molecular weight of 195.21, and is easily soluble in water, and has appropriate basicity and a certain extent of reducibility, and is safe, non-toxic. It can bind to a poorly water-soluble drug to improve the solubility of the drug in water (Mingsheng LUO, Complete collection of pharmaceutical excipients, Sichuan Science and Technology Press: 698-699), and can also complex with compounds containing phenolic hydroxyl group to protect the phenolic hydroxyl group from being oxidized.

The clinical preparation of scutellarin according to the present invention is an injection.

For the clinical preparation of scutellarin according to the present invention, the amount of the active ingredient of the injection may be 0.01g/mL∼0.03 g/mL calculated on the basis of scutellarin, i.e., the injection of scutellarin may contain 0.01g ∼ 0.03g of the active ingredient scutellarin per milliliter.

The present invention provides a method for preparing the clinical preparation comprising the following steps:
Step 1: meglumine is added into a suspension of scutellarin and stirred, to form a clear solution, the molar ratio between scutellarin and meglumine is 1.0 : 1.0∼1.5;
Step 2: the solution obtained in step 1 is cooled to -2°C ∼ 1°C, and allowed to stand for more than 8 hours, filtered through 0.45 ∼ 0.8µm filter membrane, and the filtrate is collected;
Step 3: the filtrate obtained in step 2 is formulated into a clinical preparation of scutellarin by conventional means.

In the preparation method of the clinical preparation of scutellarin according to the present invention, by forming scutellarin meglumine salt shown in Formula (I) from the scutellarin and meglumine in step 1, the solubility of scutellarin in water can be improved, and the pH value of the solution is within the range of 6.5∼6.7, in which range the phenolic hydroxy group of scutellarin is very stable, and there is no need to use organic acid, organic alkaline buffering pair to adjust a stable pH value, thus the stability of the clinical preparation of scutellarin is improved.

The clarity of the scutellarin meglumine salt is a main index reflecting the stability thereof. It is found in the present invention by the clarity test of the scutellarin meglumine salt with different pH values that, when the pH value of the solution is 6.5∼6.7, the clarities are all good, whether it is stored in the state of solution for long term or redissolved after freeze drying; and when the pH value of the solution is greater than 7.0, clarity problem occurs at different time. When the molar ratio between scutellarin and meglumine is 1.0 : 1.0∼1.5, the pH of the solution is 6.5∼6.7 which is right the most stable pH value range. Wherein, preferably, the molar ratio between scutellarin and meglumine is 1.0:1.0.

When the molar ratio between scutellarin and meglumine is 1.0 : 1.0, both of them bind completely, and reacted thoroughly to form scutellarin meglumine, when the solution concentration calculated by scutellarin is 1.0%∼3.0%, the pH is 6.5∼6.7. When the molar amout of meglumine is greater than that of scutellarin, meglumine is excessive, and because meglumine is a safe and non-toxic pharmaceutical excipient, the efficacy and the quality of the clinical preparation of scutellarin of the present invention are not affected if meglumine is present in the clinical preparation.

In the prior art, there are phenomena of difficult filtration in the process of producing the clinical preparation of scutellarin, as ingredients other than scutellarin can not be dissolved in water, and are suspended in the solution as very small particles, which are adsorbed by the filter membrane when filtration, to block the filter pore, resulting in a very slow filtration. In the preparation method of the clinical preparation of scutellarin according to the present invention, the solution obtained in step 1 is cooled to -2°C ∼ 1°C in step 2, and is allowed to stand for more than 8 hours, filtered through 0.45∼0.8 µm filter membrane, and insoluble particles suspended in the solution other than scutellarin can be well precipitated on the bottom of the solution, and the supernatant can be filtered quickly, thus the production efficiency is greatly improved.

In the preparation method of the clinical preparation of scutellarin according to the present invention, the conventional means mentioned in step 3 is specifically as the follows: the filtrate obtained is adjusted to a required concentration by adding water for injection, to obtain a total solution of scutellarin injection, which is then adsorbed by activated charcoal, filtered, filled and sterilized, to obtain the aqueous injection of scutellarin.

In the method for preparing the clinical preparations of scutellarin according to the present invention, said adsorption by activated charcoal is an adsorption by activated charcoal for injection, specifically, activated charcoal for injection is added in 0.2%∼1.0% by weight of the total solution of scutellarin injection, and adsorbed for 30∼45 minutes.

In the preparation method of the clinical preparation of scutellarin according to the present invention, the filtration is two-step filtration, first filtered through 0.45 ∼ 0.8 µm filter membrane to remove the activated charcoal for injection, then filtered through 0.2 µm filter membrane to further remove bacteria by filtration.

The preparation method of the clinical preparation of scutellarin according to the present invention further comprises steps of determining the pH value of the scutellarin injection and the content of scutellarin.

The active ingredient of the clinical preparation of scutellarin according to the present invention comprises scutellarin meglumine, which improves the water solubility and stability of scutellarin. The appearance, clarity, visible foreign matter, pH value, content, related substances, and other indicators all comply with the relevant quality requirements, proved by stress test, accelerated test and long term stability test. The stability study on the compatibility with solutions of different pH values shows that the stability for compatibility of the clinical preparation of scutellarin according to the present invention is good, suggesting that the clinical preparation of scutellarin according to the present invention is stable in nature.

The preparation method of the clinical preparation of scutellarin according to the present invention does not need to use organic acid, organic alkaline buffering pairs to adjust a stable pH value, which reduces the use of antioxidants, metal ion complexing agents and other excipients, and avoids the possibility of developing adverse effects brought by excipients to the maximal extent, thus has a high safety. In addition, the preparation method of the clinical preparation of scutellarin according to the present invention accelerates the filtration speed in the production process, and greatly improves production efficiency.

To further understand the present invention, the clinical preparation of scutellarin of the present invention and the preparation method thereof provided by the present invention will be described below in detail in combination with the examples.

### Example 1: The stability studies on the solutions of scutellarin meglumine salt, scutellarin sodium salt, scutellarin arginine salt and scutellarin lysine salt

Solutions of scutellarin meglumine salt, scutellarin sodium salt, scutellarin arginine salt and scutellarin lysine salt with the same concentration (calculated on the basis of scutellarin) were prepared and allowed to stand under the same condition, and the appearance, clarity, visible foreign matter, precipitation, and the like with time of each solution were observed. The solution of scutellarin meglumine was prepared according to the preparation method of present invention, and other solutions were prepared according to the preparation method in the prior art, and the results are shown in Table 1.

**Table 1 The stability of solutions of various composite salts of scutellarin**

| solutions | Day 0 | Day 1 | Day 2 | Day 5 | Day 10 |
|---|---|---|---|---|---|
| solution of meglumine salt | The solution was clear | The solution was clear | The solution was clear | The solution was clear | The solution was clear |
| solution of sodium bicarbonate salt | The solution was clear | about 50% is not clear | whirlpool appears | floc floating | obvious precipitation at the bottom |
| solution of arginine salt | The solution was clear | The solution was clear | the solution is clear | about 50% is not clear or whirlpool appears | floc floating |
| solution of lysine salt | The solution was clear | about 30% is not clear | whirlpool appears | floc floating | obvious precipitation at the bottom |

| | | | | | |
|---|---|---|---|---|---|
| Note: the whirlpool is a helix formed when the solution is rotated during the check of visible foreign matter due to a small amount of very fine particles in the solution precipitated at the bottom, which is obviously different from solution. | | | | | |

Results in Table 1 show that after the solution of the scutellarin meglumine salt had been placed for 10 days, the solution was still clear, and there was no phenomena of turbidity, precipitate and the like. While the solutions of scutellarin sodium bicarbonate salt, scutellarin arginine salt and scutellarin lysine salt occurred precipitation of particles of different extent after being placed for 1 day, indicating that the stability of solution of scutellarin meglumine salt is obviously superior to that of the solutions of scutellarin sodium bicarbonate salt, scutellarin arginine salt and scutellarin lysine salt.

### Example 2: The comparison test of filtration rate between solutions of scutellarin meglumine salt, scutellarin soldium salt, scutellarin arginine salt and scutellarin lysine salt

Solutions of scutellarin meglumine salt, scutellarin sodium salt, scutellarin arginine salt and scutellarin lysine salt with the same concentration (calculated on the basis of scutellarin) were prepared and were filtered by same filter membrane and filter, to conduct comparison test of filtration rates, wherein half of each solutions of scutellarin meglumine salt and scutellarin lysine salt was taken and refrigerated for more than 8 hours at -2°C ∼ 1°C as the step of the preparation method of the present invention, and the other half was not refrigerated. The other solutions were prepared according to the preparation method in the prior art, and the results are shown in Table 2.

**Table 2 The comparison of filtration rate for solutions of various scutellarin composite salts**

| solutions | filtration time of 100 mL solution filtered through 0.45µm filter membrane |
|---|---|
| meglumine salt(refrigerated) | |
| The solution was filtered smoothly, the filtration was completed in about 2 minutes. | |
| meglumine salt(unrefrigerated) | Initially filtered smoothly, and the filtration rate was slowed gradually, and filter membrane needed to be changed for 3∼4 times, and a total time of 25∼30 minutes was required. |
| arginine salt(refrigerated) | The solution was filtered smoothly, the filtration was completed in about 3 minutes. |
| arginine salt(unrefrigerated) | Initially filtered smoothly, and the filtration rate was slowed gradually, and filter membrane needed to be changed for 3∼4 times, and a total time of 25∼30 minutes was required. |
| sodium bicarbonate salt | Initially filtered smoothly, and the filtration rate was slowed gradually, and filter membrane needs to be changed for 4∼5 times, and a total time of 30∼35 minutes was required. |
| Lysine salt | Initially filtered smoothly, and the filtration rate was slowed gradually, and filter membrane needs to be changed for 3∼4 times, and a total time of 25∼30 minutes was required. |

Results in Table 2 show that, for the solutions of scutellarin meglumine salt, scutellarin sodium salt, scutellarin arginine salt and scutellarin lysine salt without refrigeration, there was no obvious difference between the filtration rate, all of them are difficult to filter, while for the solutions of scutellarin meglumine salt and scutellarin arginine salt refrigerated for more than 8 hours at -2°C ∼ 1°C as the step of the preparation method of the present invention, they could be filtered smoothly, the filtration rates were obviously accelerated, indicating that the refrigeration process according to the preparation method of the present invention is an effective method for solving the filtration difficulty of the solutions of scutellarin salts.

### Example 3: The comparison test of clarity between solutions of scutellarin meglumine salt with different pH values

Solutions of scutellarin meglumine salt with pH values of 6.5∼8.0 were prepared according to different ratios between scutellarin and meglumine, and were refrigerated at -2°C ∼ 1°C for more than 8 hours as the step of the preparation method according to the present invention, filtered through 0.45 µm filter membrane. Injections were prepared by conventional means. The injections of scutellarin meglumine salt at different pH were placed at normal temperature conditions, and were regularly sampled to test clarity. The results are shown in Table 3.

**Table 3 The comparison of clarity between solutions of scutellarin meglumine salt with different pH values**

| pH value | 0 month | 1 month | 3 months | 6 months | 12 months | 24 months |
|---|---|---|---|---|---|---|
| 6.5 | Qualified | Qualified | Qualified | Qualified | Qualified | Qualified |
| 6.9 | Qualified | Qualified | Qualified | Qualified | Qualified | Qualified |
| 7.0 | Qualified | Qualified | Qualified | Qualified | Qualified | Qualified |
| 7.1 | Qualified | Qualified | Qualified | Qualified | Unqualified | Unqualified |
| 7.5 | Qualified | Qualified | Qualified | Unqualified | Unqualified | Unqualified |
| 7.8 | Qualified | Unqualified | Unqualified | Unqualified | Unqualified | Unqualified |
| 8.0 | Qualified | Unqualified | Unqualified | Unqualified | Unqualified | Unqualified |

Results in Table 3 show that the solutions of scutellarin meglumine salt with a pH value of 6.5∼7.0 still had a very stable clarity after being stored for 24 months, while the solutions of scutellarin meglumine salt with a pH value over 7.0 all had problem of clarity at different time, indicating that pH value is an important factor affecting the clarity of solutions of scutellarin meglumine salt, and the pH value of 7.0 is a key point, the pH value of the solution of scutellarin meglumine salt should not be over 7.0.

### Example 4: Content analysis of raw scutellarin for the clinical preparation of scutellarin according to the present invention

The content of raw scutellarin for the clinical preparation of scutellarin according to the present invention is analyzed by chromatography using Agilent1100 HPLC instrument, and the content should be over 98%, to control the impurity contents of related substances from the source.

The chromatographic analysis adopted octadecylsilane-bonded silica gel as filler, and an aqueous solution of methanol-tetrahydrofuran-0.1% phosphoric acid (the ratio was 23:10:67) as mobile phase. The detection wavelength was 335 nm, and the theoretical plate number was not lower than 8000 calculated according to the peak of scutellarin. The reagents for chromatography acetonitrile and phosphoric acid were of chromatographic grade; the water for HPLC was redistilled water; hydrochloric acid and triethylamine were of analytical grade; the reference standard substance of scutellarin (Lot No.: 8420-200102) was provided by National Institutes for Drug and Biological Product Control.

An appropriate amount of scutellarin raw material was taken and precisely weighed, and dissolved by adding water and diluted to prepare a solution containing 0.1 mg (calculated in scutellarin) per 1 ml, and shaken well, and used as the solution of samples; and an appropriate amount of scutellarin reference standard substance was taken, and was dissolved by adding methanol and diluted to prepare a solution containing 0.1 mg per 1 ml, and shaken well, and used as the solution of reference standard substance; 5 µm was measured precisely and injected into the liquid chromatograph instrument, and chromatograms were recorded, the content of the raw scutellarin was obtained by external standard method calculated on peak area.

### Example 5: Preparation of scutellarin injection according to the present invention

100 g scutellarin was taken, and an appropriate amount of water for injection was added, stirred to suspend evenly, to obtain a suspension of scutellarin; 51 g meglumine was taken, and an appropriate amount of water for injection was added, stirred to dissolve it, and gradually added into the suspension of scutellarin, stirred to dissolve it completely, the solution was cooled to -2°C ∼ 1°C and refrigerated for 8 hours, and filtered through 0.45 µm filter membrane, to obtain an aqueous solution of scutellarin meglumine. The aqueous solution of scutellarin meglumine was taken, and water for injection was added to the total liquid amount, the solution concentration calculated on scutellarin was 1.0%. Activated charcoal for injection was added in the amount of 0.2% of the total solution and adsorbed for 40 minutes, and roughly filtered through 0.45 µm, and finely filtered through 0.2 µm after qualification of test for pH value and content, filled and sterilized, to obtain the injection.

### Example 6: Preparation of scutellarin injection according to the present invention

200 g scutellarin was taken, and an appropriate amount of water for injection was added, stirred to suspend evenly, to obtain a suspension of scutellarin; 85 g meglumine was taken, and an appropriate amount of water for injection was added, stirred to dissolve it, and gradually added into the suspension of scutellarin, stirred to dissolve it completely, the solution was cooled to -2°C ∼ 1°C and refrigerated for 8 hours, and filtered through 0.8 µm filter membrane, to obtain an aqueous solution of scutellarin meglumine. The aqueous solution of scutellarin meglumine was taken, and water for injection was added to the total liquid amount, the solution concentration calculated on scutellarin was 2.0%. Activated charcoal for injection was added in the amount of 0.5% of the total solution and adsorbed for 45 minutes, and roughly filtered through 0.8 µm, and finely filtered through 0.2 µm after qualification of test for pH value and content, filled and sterilized, to obtain the injection.

### Example 7: Preparation of scutellarin injection according to the present invention

300 g scutellarin was taken, and an appropriate amount of water for injection was added, stirred to suspend evenly, to obtain a suspension of scutellarin; 189 g meglumine was taken, and an appropriate amount of water for injection was added, stirred to dissolve it, and gradually added into the suspension of scutellarin, stirred to dissolve it completely, the solution was cooled to -2°C ∼ 1°C and refrigerated for 8 hours, and filtered through 0.45 µm filter membrane, to obtain an aqueous solution of scutellarin meglumine. The aqueous solution of scutellarin meglumine was taken, and water for injection was added to the total liquid amount, the solution concentration calculated on scutellarin was 3.0%. Activated charcoal for injection was added in the amount of 1.0% of the total solution and adsorbed for 45 minutes, and roughly filtered through 0.45 µm, and finely filtered through 0.2 µm after qualification of test for pH value and content, filled and sterilized, to obtain the injection.

### Example 8: The stability study on the clinical preparation of scutellarin according to the present invention

Comprehensive investigation into stability, including stress test, accelerated test, and long term stability test was performed on the injection prepared according to Example 7 of the present invention. The conditions for each stability investigation and the method for testing visible foreign matter all referred to the related regulations in the appendix of Chinese Pharmacopoeia. The stress test was performed for 10 days, the accelerated test was performed for 6 months, and the long-term stability test was performed for 24 months. The results are shown in Table 4, Table 5 and Table 6.

**Table 4 stress test**

| | | appearance | visible foreign matter | pH value | content (%) |
|---|---|---|---|---|---|
| Day 0 | | light brown clear solution | conform with the regulations of Chinese Pharmacopoeia | 6.8 | 100 |
| high temperature (60°C) | Day 5 | light brown clear solution | conform with the regulations of Chinese Pharmacopoeia | 6.7 | 99.8 |
| | Day 10 | light brown clear solution | conform with the regulations of Chinese Pharmacopoeia | 6.8 | 100.1 |
| Light (4500±500LX) | Day 5 | light brown clear solution | conform with the regulations of Chinese Pharmacopoeia | 6.6 | 99.7 |
| | Day 10 | light brown clear solution | conform with the regulations of Chinese Pharmacopoeia | 6.9 | 99.6 |

| | | | | | |
|---|---|---|---|---|---|
| Note: For the injection, test under a condition of high humidity is not necessary to be performed; the content at day 0 was counted as 100%. | | | | | |

**Table 5 Accelerated test**

| | appearance | visible foreign matter | related substances | pH value | content (%) |
|---|---|---|---|---|---|
| 0 month | light brown clear solution | conform with the regulations of Chinese Pharmacopoeia | conform with the quality regulations | 6.8 | 99.8 |
| 1 month | light brown clear solution | conform with the regulations of Chinese Pharmacopoeia | conform with the quality regulations | 6.9 | 99.6 |
| 2 months | light brown clear solution | conform with the regulations of Chinese Pharmacopoeia | conform with the quality regulations | 6.7 | 99.9 |
| 3 months | light brown clear solution | conform with the regulations of Chinese Pharmacopoeia | conform with the quality regulations | 6.6 | 99.8 |
| 6 months | light brown clear solution | conform with the regulations of Chinese Pharmacopoeia | conform with the quality regulations | 6.8 | 99.7 |

| | | | | | |
|---|---|---|---|---|---|
| Note: The temperature was 40°C±2°C, and the relative humidity was 75%±5%. | | | | | |

**Table 6 long-term stability test**

| | appearance | visible foreign matter | related substances | pH value | Content (%) |
|---|---|---|---|---|---|
| 0 month | light brown clear solution | conform with the regulations of Chinese Pharmacopoeia | conform with the quality regulations | 6.8 | 99.8 |
| 3 months | light brown clear solution | conform with the regulations of Chinese Pharmacopoeia | conform with the quality regulations | 6.7 | 100.2 |
| 6 months | light brown clear solution | conform with the regulations of Chinese Pharmacopoeia | conform with the quality regulations | 6.6 | 99.9 |
| 9 months | light brown clear solution | conform with the regulations of Chinese Pharmacopoeia | conform with the quality regulations | 6.7 | 99.6 |
| 12 months | light brown clear solution | conform with the regulations of Chinese Pharmacopoeia | conform with the quality regulations | 6.9 | 99.8 |
| 18 months | light brown clear solution | conform with the regulations of Chinese Pharmacopoeia | conform with the quality regulations | 6.8 | 99.7 |
| 24 months | light brown clear solution | conform with the regulations of Chinese Pharmacopoeia | conform with the quality regulations | 6.6 | 99.7 |

| | | | | | |
|---|---|---|---|---|---|
| Note: The temperature was 25°C±2°C, and the relative humidity was 60%±10% | | | | | |

It can be known from the results in Table 4, Table 5 and Table 6 that there is no obvious change with respect to the main quality indicators of the scutellarin injection prepared in Example 7 of the present invention after being placed under severe conditions of high temperature, strong light for 10 days, being placed under an accelerated condition of 40°C and 75% relative humidity for 6 months, being placed at a condition of 25°C± 2°C and a relative humidity of 60% + 10% for 24 months, suggesting that the clinical preparation of scutellarin according to the present invention is stable and controllable in quality.

## Claims

1. A clinical solution preparation of scutellarin, **characterized in that** it comprises active ingredients and water for injection, and the active ingredient comprises a compound shown in Formula (I) 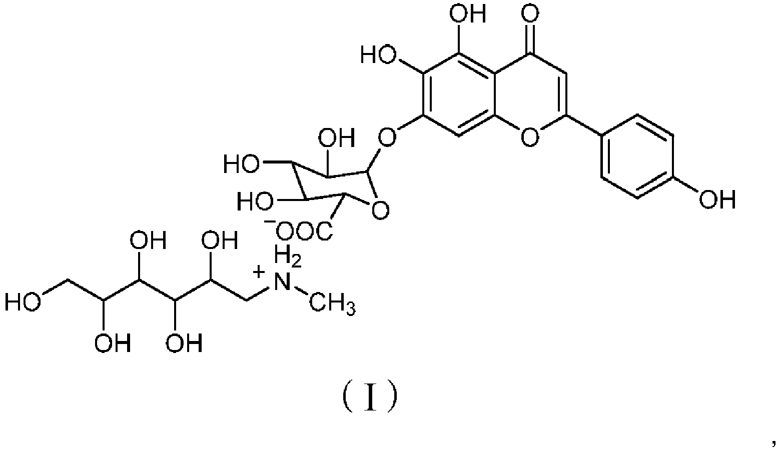 wherein the molar ratio between scutellarin and meglumine is 1.0 : 1.0, and the pH of the preparation is 6.5∼6.7.

2. The clinical solution preparation according to claim 1, **characterized in that** the clinical preparation is an injection.

3. The clinical solution preparation according to claim 2, **characterized in that** the amount of the active ingredient of the injection is 0.01 g/mL∼0.03g/mL calculated on the basis of scutellarin.

4. A method for preparing the clinical solution preparation of scutellarin according to any of claims 1∼3, comprising:
| | |
|---|---|
| Step 1: | meglumine is added into a suspension of scutellarin and stirred, to form a clear solution, wherein the molar ratio between scutellarin and meglumine is 1.0 : 1.; |
| Step 2: | the solution obtained in step 1 is cooled to -2°C ∼ 1°C, and allowed to stand for more than 8 hours, filtered through 0.45 ∼ 0.8µm filter membrane, and the filtrate is collected; |
| Step 3: | the filtrate obtained in step 2 is formulated into a clinical solution preparation of scutellarin by conventional means. |

5. The method according to claim 4, **characterized in that** the clinical solution preparation is an injection.

## Patentansprüche

1. Klinisches Lösungspräparat aus Scutellarin, **dadurch gekennzeichnet, dass** es Wirkstoffe und Wasser für Injektionen aufweist, und der Wirkstoff eine Verbindung aufweist, die in Formel (I) dargestellt ist 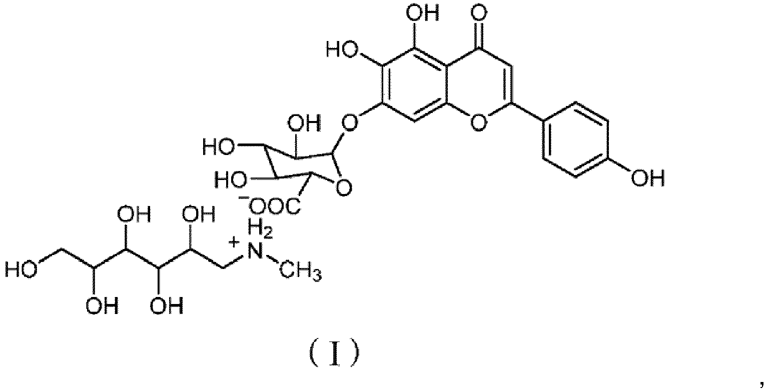 wobei das Molverhältnis zwischen Scutellarin und Meglumin 1,0 : 1,0 beträgt, und der pH-Wert des Präparats 6,5 ∼ 6,7 beträgt.

2. Klinisches Lösungspräparat nach Anspruch 1, **dadurch gekennzeichnet, dass** das klinische Präparat eine Injektion ist.

3. Klinisches Lösungspräparat nach Anspruch 2, **dadurch gekennzeichnet, dass** die Wirkstoffmenge der Injektion 0,01 g/mL∼0,03g/mL beträgt, berechnet auf der Grundlage von Scutellarin.

4. Verfahren zur Herstellung des klinischen Lösungspräparats aus Scutellarin nach einem der Ansprüche 1∼3, umfassend:
| | |
|---|---|
| Schritt 1: | Meglumin wird in eine Scutellarin-Suspension gegeben und gerührt, um eine klare Lösung zu bilden, wobei das Molverhältnis zwischen Scutellarin und Meglumin 1,0 : 1, beträgt; |
| Schritt 2: | die in Schritt 1 erhaltene Lösung wird auf -2°C ∼ 1°C gekühlt, und für mehr als 8 Stunden stehen gelassen, gefiltert durch 0,45 ∼ 0,8 µm Filtermembran, und das Filtrat wird aufgefangen; |
| Schritt 3: | das in Schritt 2 erhaltene Filtrat wird auf herkömmliche Weise in ein klinisches Lösungspräparat aus Scutellarin formuliert. |

5. Verfahren nach Anspruch 4, **dadurch gekennzeichnet, dass** das klinische Lösungspräparat eine Injektion ist.

## Revendications

1. Préparation clinique de scutellarine en solution, **caractérisée en ce qu'**elle comprend des principes actifs et de l'eau pour injection, et **en ce que** le principe actif comprend un composé représenté par la Formule (I) 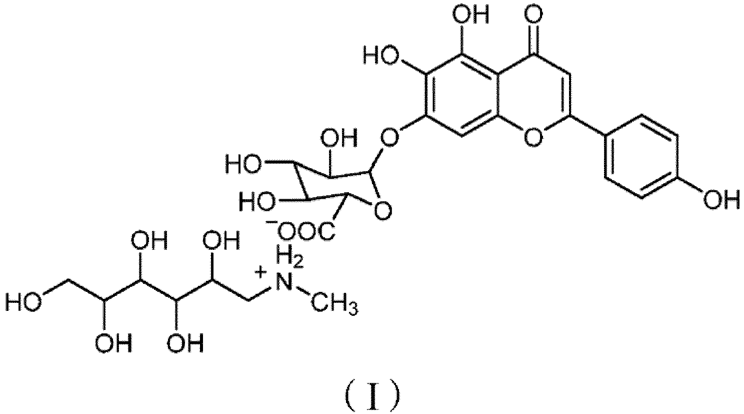 dans laquelle le rapport molaire entre la scutellarine et la méglumine est égal à 1,0 : 1,0, et le pH de la préparation est compris entre 6,5 et 6,7.

2. La préparation clinique en solution selon la revendication 1, **caractérisée en ce que** la préparation clinique est une injection.

3. La préparation clinique en solution selon la revendication 2, **caractérisée en ce que** la quantité du principe actif de l'injection est comprise entre 0,01 g/mL et 0,03 g/mL, calculée sur la base de la scutellarine.

4. Procédé de préparation de la préparation clinique de scutellarine en solution selon l'une quelconque des revendications 1-3, le procédé comprenant :
| | |
|---|---|
| Étape 1 : | de la méglumine est ajoutée à une suspension de scutellarine puis agitée, afin de former une solution claire, le rapport molaire entre la scutellarine et la méglumine étant égal à 1,0 : 1, ; |
| Étape 2 : | la solution obtenue à l'étape 1 est refroidie jusqu'à -2 °C ∼ 1 °C et on la laisse reposer pendant plus de 8 heures, ensuite elle est filtrée à travers une membrane de filtre de 0,45 ∼ 0,8 µm, et le filtrat est collecté ; |
| Étape 3 : | le filtrat obtenu à l'étape 2 est formulé comme une préparation clinique de scutellarine en solution à l'aide de moyens conventionnels. |

5. Le procédé selon la revendication 4, **caractérisé en ce que** la préparation clinique en solution est une injection.
